(19) Europäisches Patentamt
European Patent Office
Office européen des brevets

(11) Publication number: **0 045 990**
**A1**

(12) # EUROPEAN PATENT APPLICATION

(21) Application number: **81200869.6**

(22) Date of filing: **03.08.81**

(51) Int. Cl.³: **C 07 D 403/06**
**A 61 K 31/505**

(30) Priority: **07.08.80 GB 8025832**

(43) Date of publication of application:
**17.02.82 Bulletin 82/7**

(84) Designated Contracting States:
**AT DE GB NL SE**

(71) Applicant: **Farmatis S.r.l.**
**Corso Europa n. 5**
**I-20100 Milano 1(IT)**

(72) Inventor: **Grey, Paul Ouranovsky**
**137 Harley Street**
**London W 1(GB)**

(74) Representative: **Dragotti, Gianfranco et al,**
**SAIC BREVETTI S.a.s. di Ing. G. Dragotti & C. Via**
**Spontini, 1**
**I-20131 Milano(IT)**

(54) **Novel antibacterial and antiprotozoal derivatives, a process for the preparation thereof, and related pharmaceutical compositions.**

(57) The novel derivatives, having the general formula:

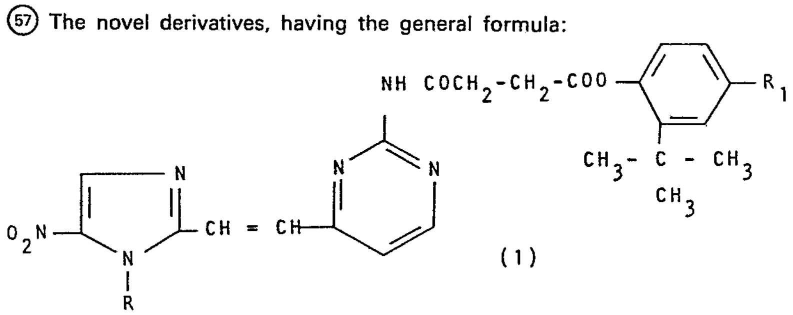

(1)

wherein R is a lower alkyl containing 1 to 6 carbon atoms and $R_1$ is H or $-OCH_3$, are useful therapeutical agents against protozoal diseases and bacterial infections, particularly as trichomonacidal agents.

EP 0 045 990 A1

"Novel antibacterial and antiprotozoal derivatives, a process for the preparation thereof and related pharmaceutical compositions"

oooooooooo

The present invention relates to novel compounds, active against protozoal diseases and bacterial infections, having the following general formula:

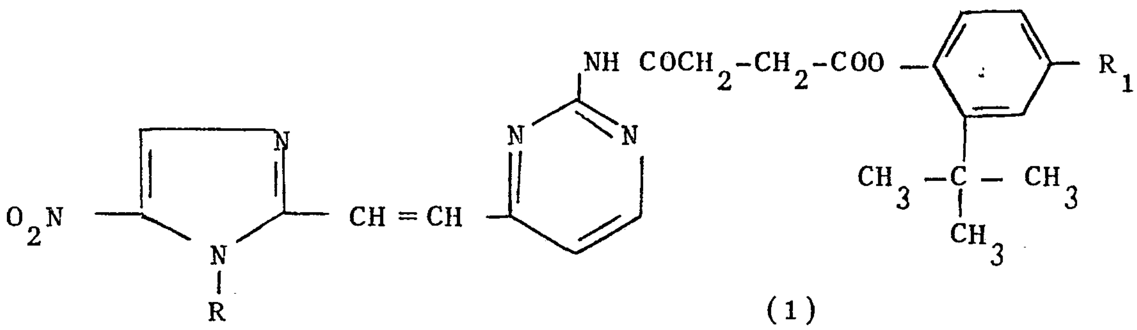

(1)

wherein R is a lower alkyl, containing 1 to 6 carbon atoms, preferably 1 to 3 carbon atoms, and $R_1$ is H or $-OCH_3$.

More particularly, the present invention relates to the p-methoxy-o-tert-butyl-phenol ester of 5-nitro-imidazolyl-2-ethenyl-(2-amino-pyrimidyl)-N-succinic acid useful in the treatment of diseases caused by protozoa and bacteria, as for instance trichomoniasis, in its various sites of infection, amoebiasis, giardiasis and anaerobic bacterial infections in their sites of infection.

It is well known that trichomoniasis is a social disease that affects both women and men in a high percentage.

In women, it occurs as a form of vaginitis in most cases.

In men, it occurs asymptomatically and may cause sterility.

Remarkable efforts have been made in order to find out a treatment for this disease that holds the first rank among veneral infections and several attempts have been made in order to discover effective and safe chemotherapic agents.

The ideal drug should be selectively effective on Trichomonas vaginalis, but not on Döderlein's bacillus, nor on Meisseria gonorrheae, in order not to upset the vaginal ecology and in order not to mask with an unsuitable treatment the combined gonorrheal infection.

Moreover, the ideal chemotherapic drug should not interfere with alcohol metabolism

(antabuse-like effect).

The metronidazole, namely 1-(2-hydroxyethyl)-2-methyl-5-nitro-imidazole, (Jacobs et al, USP 2,944,061) is still now an available and frequently prescribed drug for the treatment of trichomoniasis, although it is not completely satisfactory, since certain strains of Trichomonas vaginalis became resistant to this drug.

Other proposals include the use of certain 1-lower alkyl-2-(2-substituted-vinyl)-5-nitroimidazoles, as an intermediate to sulfur-containing antiprotozoal agents (Miller et al, USP 3,549, 626), and the use of certain 2-(5-nitro-2-furyl)-vinyl pyrimidine derivatives (Minami et al, 3,464,982).

USP No. 3,882,105, herein included by reference, disclose and claims the use of 2-amino-4- 2-(1-lower alkyl-5-nitro-2-imidazolyl)-vinyl -pyrimidine for the treatment of protozoal diseases and bacterial infections, particularly as anti-trichomonal agent.

These compounds, (also known under the common name of P-compounds), correspond to the following formula:

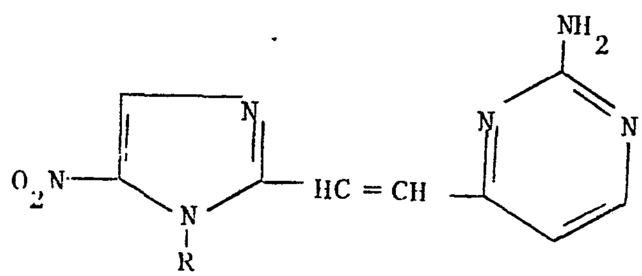

(2)

wherein R is a lower alkyl of 1 to 6, preferably 1 to 3 carbon atoms.

Other compounds, (also known as O-compounds), have been further proposed, which are the 3-N-oxides of the P-compounds, having the formula:

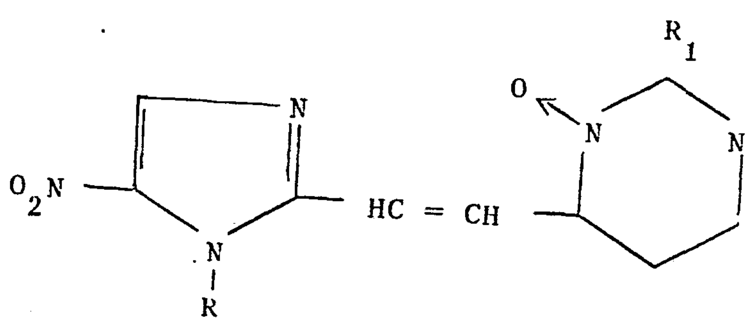

(3)

wherein R is a lower alkyl, which may be substituted with hydroxy or a lower alkyl

sulfonyl group, and $R_1$ is $-NH_2$ or -NH-C-H.

According to the present invention there is provided a compound of formula (I) in which $R_1$ is $-OCH_3$ and R is $CH_3$, which is a crystalline yellow compound, which decomposes upon heating to 170°C, is soluble (1 : 100) in methanol and (1 : 30) in dimethylformamide, but insoluble in water.

For the preparation of the novel compounds above referred to, the process of the present invention provides the steps of (a) preparing the N-succinyl derivative of the 2-amino-4- 2-(1-lower alkyl-5-nitro-2-imidazolyl)-vinyl -pyrimidine by refluxing this compound with succinic anhydride and then reacting the N-succinyl derivative with o-tert butyl-phenol, possibly substituted in the para position with lower alcoxy, in the presence of concentrated mineral acid.

The following example illustrates, without limiting purpose, the process of the invention.

## EXAMPLE

24.6 g (0.1 moles) of azanidazole, i.e. 2-amino-4- 2-(1-methyl-5-nitro-2-imidazolyl)-vinyl pyrimidine in 500 mls of dimethylformamide are boiled to reflux for 3 hours with 10.2 g (0.1 moles) of succinic anhydride.

It is preferable to use an excess of anhydride of 2-3% b.w.

The reaction mixture, as a clear solution, is cooled and then poured onto 500 g of ground ice.

A yellow, crystalline mass is precipitated, which is the N-succinyl derivative of azanidazole.

The purification of the reaction product is effected by taking it with a 10% $NaH\ CO_3$ solution and precipitating again with diluted HCl (1 : 1).

The yield of the reaction product is 80% of the theoretical value, and it has no definite melting point but decomposes at 150°C.

The analysis of the reaction product by IR and NMR corresponds to N-succinyl derivative of azanidazole.

1 mole of this derivative in 50 times its weight of dimethylformamide is refluxed with 1 mole of p-methoxy-o-tert-butyl-phenol, in the presence of 2 mls of concentrated $H_2SO_4$, for 6 hours.

The reaction mixture is distilled under reduced pressure (20 mm Hg), thus removing

about one half of the solvent, and then the desired compound, i.e. p-methoxy-o-tert-butyl-phenol ester of 5-nitro-imidazolyl-2-ethenyl-(2-amino-pyrimidyl)-N-succinic acid, is crystallized with a yield of 70% of the theoretical value, the product being decomposed at about 170°C.

The compounds of the present invention, particularly the derivative disclosed in the example have been experimentally tested, from the microbiological, pharmacological and clinical point of view.

Actions

The novel compounds possess direct trichomonacidal and amoebacidal activity against Trichomonas vaginalis and Entamoeba histolitica, and bactericidal activity against anaerobic microorganisms.

The novel compounds are usually well absorbed after oral administration, and well tolerated.

Indications

- Symptomatic and asymptomatic trichomoniasis in females and in males
- Acute intestinal amoebiasis and amoebic liver abscess
- Anaerobic bacterial infections at sites such as abdomen, pelvis and chest, and in the treatment of bacteremia, endocarditis, and meningitis. For the prophylaxis in surgical operations in which the risk of postoperative anaerobic infections is high.

Microbiology (in vitro - in vivo)

- The novel compounds are active in vitro against Trichomonas vaginalis at concentrations lower than those of metronidazole on the sensitive strains, and active on several strains which are resistant to metronidazole.
- The killing-rate of the novel compounds is higher than that of metronidazole.
- The novel compound's are active in vitro against Entamoeba histolitica at concentrations of the same range of the metronidazole.
- The novel compounds are active in vitro against anaerobic microorganisms at concentrations lower than those of metronidazole on the sensitive strains, and active on several strains which are resistant to metronidazole.
- The novel compounds are not active in vitro against Dôderlein's bacillus
- The novel compounds are not active in vitro against Neisseria gonorrheae.

The *in vivo* efficacy of the novel compounds administered in animals by oral route in experimental infections is higher than that of metronidazole.

Pharmacokinetics

- In animals the novel compounds administered by oral route are rapidly absorbed: a single dose of 500 mg/kg results in 3 mcg/ml blood levels 1 h after the oral administration in rats, the same dose/kg in 20 mcg/ml blood levels 3 hrs after oral administration in dog.
- The half-life of novel compounds administered in animal (dog) and in man is shorter than that of metronidazole (movelc compounds 1h, metronidazole 7.3 hrs.)

Biochemistry

- Differently from metronidazole the novel compounds do not interfere in the alcohol metabolism.
- The novel compounds increase the activity of the hepatic glutatione-S-transferase (a group of multifuncional detoxification proteins), while metronidazole does not exert this action.

Toxicity

The acute toxicity ($LD_{50}$) of the novel compounds is 1250 mg/kg by oral route in mice.

Clinical investigation

The clinical investigation involved 42 patients, aged from 22 to 55, affected by vaginitis induced by Trichomonas Vaginalis.

The diagnosis was determined through bacterioscopic, cultural and May-Grunwald Giemsa examinations, which were repeated 3 days after the end of the treatment.

The 42 Patients were splitted into 6 groups of 7 patients each.

Here follow the most effective dosage schedule and single and daily doses in humans:

1 st group: 7 patients aged from 22 to 54
scheme of treatment: 2 capsules of 200 mg
each per day for 3 days.

2nd group: 7 patients aged from 35 to 48
scheme of treatment: 2 capsules of 200 mg
each per day for 5 days.

3rd group: 7 patients aged from 40 to 55
scheme of treatment: 9 capsules of 200 mg each in three administrations, in 24 hour. span, i.e. 3 capsules at noon, 3 capsules in the evening and 3 capsules at midday of the following day.

4th group 7 patiens aged from 25 to 40
scheme of treatment: single dose administration; 9 capsules of 200 mg each

5th group: 7 patiens aged from 30 to 45
local treatment: 3 vaginal tablets of 200 mg each per day for 5 day.

6th group: 7 patients aged from 40 to 55
combined (local and oral) treatment at following scheme: 2 capsules of 200 mg each per os per day for 5 days 1 vag. tab. of 200 mg twice a day for 5 days.

The drug turned out to be well tolerated and highly active against Trichomoniasis. Complete success was obtained in 25 patiens out of 28, treated orally (1st, 2nd, 3rd, 4th groups), i.e. 98%, without any significant difference among the groups.

In the 5th group, treated only locally, Trichomonas vaginalis disappeared in 6 out of 7 patients, i.e. 85% of complete recovery.

The 6th group, treated both orally andlocally, registered 100% of positive results.

Briefly summarizing:

many are the advantages that the compounds of the present invention have over metronidazole;

- a higher in vitro activity on Trichomonas vaginalis strains that are metronidazole sensitive
- The in vitro activity on metronidazole resistant strains
- a higher effectiveness on experimental infections
- a shorter half-life, that means less risks of toxicity
- lack of interference with alcohol metabolism, so that alcoholic drinks can be taken during the treatment, while this is not advisable during treatment with metronidazole
- the activation of certain multifunctional detoxification proteins

- a more favourable dosage schedule: lower daily dose, shorter period of t treatment, lower amount of drug totally administered.

For the oral admiistration, the active compound is prepared in form of pills, tablets or capsules with a suitable carrier. Unit dosages of 50 to 500 mg of active compound are satisfactory, these unit dosages containing the normal diluents, excipients and lubricants: suitable carriers are, for example, solid cubstances, such as lactose, magnesium stearate, starch, gelatin, agar.

Alternatively, the active compound can be suspended or dissolved in a suitable liquid medium.

The final drug can be in form of a solution, emulsion, suspension, syrup.

Liquid carriers which can be used include sesame oil, olive oil and water. For topical treatments, gels, creams, ointments or suppositories are generally used.

Vaginal inserts containing, as inactive ingredients, lactose, polyvinyl alcohol or other water soluble, non toxic, polymers, are commonly used and generally supplemented with an oral treatment.

For parenteral injection, the active compound is contained in a suitable sterile solution.

## CLAIMS

1) Novel compouns having the following general formula:

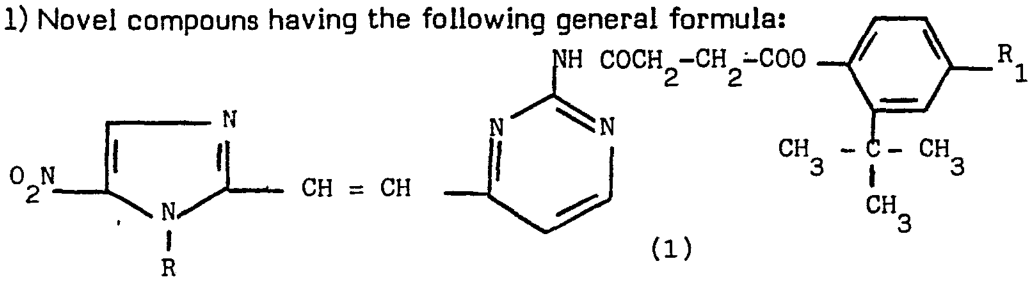

(1)

wherein R is a lower alkyl containing 1 to 6 carbon atoms, preferably 1 to 3 carbon atoms, and $R_1$ is H or $-OCH_3$.

2) p-methoxy-o-tert-butyl-phenol ester of 5-nitro-imidazolyl-2-ethenyl-(2-amino-pyrimidil)-N-succinic acid.

3) A process for the preparation of the novel compounds of claim 1, comprising the steps of:

(a) preparing the N-succinyl derivative of the 2-amino-4- 2-(1-lower alkyl-5-nitro-2-imidazolyl)-vinyl -pyrimidine by refluxing the latter with succinic anhydride, and

(b) reacting the thus obtained N-succinyl derivative with o-tert-butyl-phenol, possibly substituted in the para position with lower alkoxy, in the presence of concentrated mineral acid.

4) A process according to claim 3, wherein said step (a) is carried out in dimethylformamide, the succinic anhydride is in a slinght excess and the refluxing is continued for 3 hours.

5) A process according to claim 4, wherein said excess is 2-3%.

6) A process according to claim 3, wherein said mineral acid is sulfuric acid and the reaction step (b) is continued for 6 hours.

7) A process according to claim 3, wherein 2-amino-4- 2-(1-methyl-5-nitro-2-imidazolyl)-vinyl -pyrimidine is boiled to reflux in dimethylformamide with succinic anhydride, and the N-succinyl derivative is refluxed with p-methoxy-o-tert-butyl-phenol in the presence of concentrated sulfuric acid, the reaction mixture being then distilled under reduced pressure and the desired reaction product being then crystallized.

8) o-tert-butyl phenol ester of 5-nitro-imidazolyl-2-ethenyl-(2-amino-pyrimidil)-N-succinic acid, whenever prepared according to the process of claims3 to 6.

9) p-methoxy-o-tert-butyl-phenol ester of 5-nitro-imidazolyl-2-ethenyl-(2-amino-pyrimidyl)-N-succinic acid, whenever prepared according to the process of claim 7.

10) A pharmaceutical composition for the treatment of protozoal diseases and bacterial infections, containing, as the active ingredient, a compound according to claim 1, together with a suitable carrier.

11) A pharmaceutical composition according to claim 10, for topical treatment in form of cream, ointment, emulsion or gel.

12) A pharmaceutical composition according to claim 10, in form adapted for parenteral administration, together with a liquid carrying medium.

13) A pharmaceutical composition according to claim 10, for oral administration, in form of pills, tablets or capsules, together with a solid carrier.

14) A pharmaceutical composition according to claim 10, for oral administration, in form of syrup.

15) A pharmaceutical composition according to the claim 10 to 14, useful as trichomonacidal agent.

0045990

European Patent Office

# EUROPEAN SEARCH REPORT

Application number

EP 81200869.6

| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| | US - A - 3 969 520 (GARZIA)<br>* Column 12, lines 5-24 *<br>---- | 1,10-15 | C 07 D 403/06<br>A 61 K 31/505 |
| | | | TECHNICAL FIELDS SEARCHED (Int. Cl.3) |
| | | | C 07 D 403/00<br>A 61 K 31/00 |
| | | | CATEGORY OF CITED DOCUMENTS |
| | | | X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| X | The present search report has been drawn up for all claims | | &. member of the same patent family, corresponding document |

| Place of search | Date of completion of the search | Examiner |
|---|---|---|
| VIENNA | 30-10-1981 | BRUS |

EPO Form 1503.1 06.78